(19) Europäisches Patentamt
European Patent Office
Office européen des brevets

(11) Publication number: **0 331 422 A2**

(12) **EUROPEAN PATENT APPLICATION**

(21) Application number: 89301963.8

(22) Date of filing: 28.02.89

(51) Int. Cl.4: C07C 45/64 , C07C 45/65 , C07C 49/825 , C07C 49/83 , C07C 49/84 , C07C 45/00 , C07C 45/46 , C07C 49/403

(30) Priority: 02.03.88 JP 47515/88
02.03.88 JP 47516/88

(43) Date of publication of application:
06.09.89 Bulletin 89/36

(84) Designated Contracting States:
AT BE CH DE ES FR GB GR IT LI LU NL SE

(71) Applicant: MITSUI PETROCHEMICAL INDUSTRIES, LTD.
2-5, Kasumigaseki 3-chome Chiyoda-ku
Tokyo 100(JP)

(72) Inventor: Hirowatari, Noriyuki
Mitsui Petrochemical Ind. Ltd. 1-2, Waki 6-chome
Waki-cho Kuga-gun Yamaguchi(JP)
Inventor: Furuya, Yoshiaki
Mitsui Petrochemical Ind. Ltd. 1-2, Waki 6-chome
Waki-cho Kuga-gun Yamaguchi(JP)

(74) Representative: Myerscough, Philip Boyd et al
J.A.Kemp & Co. 14, South Square Gray's Inn
London, WC1R 5EU(GB)

(54) **Method of preparing 2-acylresorcinols.**

(57) A method of preparing a 2-acylresorcinol which comprises dehydrogenating a 2-acyl-1,3-cyclohexanedione of the general formula

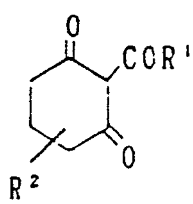

wherein $R^1$ represents an alkyl, aralkyl or aryl group, and $R^2$ represents hydrogen, or an alkyl, alkoxy or acylamino group, in the presence of a dehydrogenation catalyst (such as a Group VIII metal) or a dehydrogenation agent (such as sulphur or selenium) at an elevated temperature, to provide a 2-acylresorcinol of the general formula

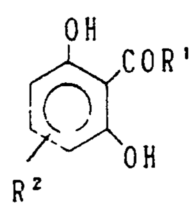

EP 0 331 422 A2

wherein $R^1$ and $R^2$ are as defined.

## Method of Preparing 2-Acylresorcinols

This invention relates to a method of preparing 2-acylresorcinols, more particularly to a method of preparing 2-acylresorcinols by a single step dehydrogenation of 2-acyl-1,3-cyclohexanediones.

2-Acylresorcinols are useful as intermediates for the production of a variety of pharmaceuticals and agricultural chemicals. For example, 2-acetylresorcinol is used for the production of intermediates for cough and phlegm medicines.

Many methods are already known to prepare 2-acetyl-resorcinol. For instance, a method is known wherein resorcinol and ethyl acetoacetate are used as starting materials, as described in Organic Syntheses, Coll. Vol. 3, p. 281 (1965), but this method needs four steps. There are also known a method in which 2-acetyl-1,3-cyclohexanedione is chlorinated to provide 2-acetyl-2-chloro-1,3-cyclohexane dione, and then the product is treated with hydrogen chloride in dimethylformamide, as described in Tetrahedron, 29, 3857 (1973). However, this method provides 2-acetylresorcinol only in a low overall yield of about 50 %. A further method is also known in which methyl β-resorcylate is produced as an intermediate, as described in J. Ind. Chem. Soc, 28, 245 (1951). As described above, these prior methods need many reaction steps and the yields are not so high.

Catalytic reduction of 2-acetyl-1,3-cyclohexanedione at normal temperatures and pressures in the presence of Pd on activated carbon is also already known, as described in J. Chem. Soc., 1953, 803, wherein 2-acetylcyclohexanone is reportedly a main reaction product accompanied by a small amount of 2-ethyl-1,3-cyclohexanedione. On the other hand, direct acetylation of resorcinol in the presence of anhydrous aluminum chloride has been found to provide 2-acetylresorcinol in less than one percent yield. Thus, there has been known no method of preparing 2-acylresorcinols by a single step dehydrogenation of 2-acyl-1,3-cyclohexanediones.

It is, therefore, an object of this invention to provide a method of preparing high purity substituted or unsubstituted 2-acylresorcinols inclusive of 2-acetylresorcinol in high yields by a single step dehydrogenation of 2-acyl-1,3-cyclohexanediones.

There is provided in accordance with this invention a method of preparing 2-acylresorcinols which comprises: dehydrogenating a 2-acyl-1,3-cyclohexanedione represented by the general formula of

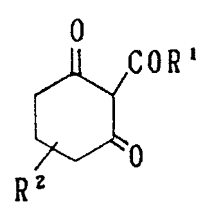

(I)

wherein R′ represents an alkyl, aralkyl or aryl group, and $R^2$ represents a hydrogen, alkyl, alkoxy or acylamino group, in the presence of a dehydrogenation catalyst or a dehydrogenation agent, at elevated temperatures, to provide a 2-acylresorcinol represented by the general formula of

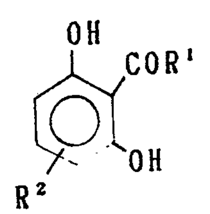

(II)

wherein $R^1$ and $R^2$ are the same as stated above.

The starting material used in the method of this invention is represented by the above general formula (I), in which $R^1$ is an alkyl preferably of 1-6 carbons, an aralkyl preferably such as benzyl, or an aryl

preferably such as phenyl, and $R^2$ is a hydrogen, an alkyl preferably of 1-6 carbons, an alkoxy wherein the alkyl has preferably 1-6 carbons, or an acylamino group in which the alkyl has preferably 1-6 carbons.

Therefore, preferred 2-acyl-1,3-cyclohexanediones used as a starting material include, for example, 2-acetyl-1,3-cyclohexanedione, 2-propionyl-1,3-cyclohexanedione, 2-benzoyl-1,3-cyclohexanedione, 2-acetyl-4-ethyl-1,3-cyclo-hexanedione and 2-acetyl-4-acetylamino-1,3-cyclohexanedione.

The dehydrogenation of such 2-acyl-1,3-cyclohexanediones is carried out in the presence of either a dehydrogenation catalyst or a dehydrogenation agent. The catalyst usable includes VIII group metals such as Ni, Rh, Pt, Pd or Ir, and these metals are used singly or as a mixture of two or more. The metal may be used as it is, however, it is preferably supported on a carrier material, such as activated carbon, alumina, titania, silica-alumina, zeolite, magnesia, or a mixture of two or more of these. When the catalyst is composed of a metal supported on a carrier material, the catalyst contains a metal usually in amounts of about 0.01-15 % by weight, preferably in amounts of about 0.5-10 % by weight. Among the metal/carrier catalysts, Pd/activated carbon or Rh/alumina is most preferred since such a catalyst provides 2-acylresorcinols especially in high yields.

The catalyst is used usually in amounts of about 0.01-1 parts by weight, preferably in amounts of about 0.05-0.5 parts by weight, in relation to one part by weight of a 2-acyl-1,3-cyclohexanedione used. When the catalyst is composed of a metal supported on a carrier material, the catalyst is defined as a metal and a carrier supporting the metal.

The dehydrogenation may also be effected using a known dehydrogenation agent which is an element or a compound capable of reacting with hydrogen, and preferred agents may be exemplified by sulfur or selenium. The dehydrogenation agent is used usually in amounts of about 1-10 moles, preferably in amounts of about 1.1-3 moles, in relation to one mole of a 2-acyl-1,3-cyclohexanedione used. The dehydrogenation catalyst and agent may be used together, if desired.

When either a dehydrogenation catalyst or an agent is used, the dehydrogenation is carried out usually in the presence of an organic solvent inactive to the reaction. Preferred solvents include, for example, polyethylene glycol and its mono- or dialkyl ether such as tetraethylene glycol dimethyl ether, triethylene glycol dimethyl ether, diethylene glycol monoethyl ether and diethylene glycol monomethyl ether. Aromatic ethers such as diphenyl ether or higher alcohols such as 1-octanol are also usable as a solvent.

The dehydrogenation of a 2-acyl-1,3-cyclohexanedione is carried out while hydrogen generated in the reaction is removed continuously from the reaction mixture. By way of example, the reaction is carried out in the presence of 2-propanol, and if desired under a nitrogen stream, and the hydrogen generated in the reaction is removed continuously with the 2-propanol vapor and/or nitrogen. As a second method, the hydrogen generated in the reaction may be reacted with and trapped by a hydrogen acceptor which has been in advance added to the reaction mixture. The hydrogen acceptor may be, for example, α-methylstyrene or nitrobenzene. The former method is generally preferred, while the latter method may be useful when a dehydrogenation catalyst is used in the reaction.

The dehydrogenation is carried out usually at temperatures of about 100-250 °C over a period of about 0.5-20 hours, although not limited thereto. When a catalyst is used, the reaction may be effected at relatively low temperatures, preferably at temperatures of about 150-200 °C, whereas when a dehydrogenation agent is used, it is preferred that the reaction is carried out at relatively high temperatures, for example at 200-250 °C.

In carrying out the dehydrogenation of a 2-acyl-1,3-cyclohexanedione, the starting material may be fed as a solution in, for example, 2-propanol, continuously over a time into a reaction vessel which contains a catalyst. As an another method, a mixture of a 2-acyl-1,3-cyclohexane-dione and a dehydrogenation agent may be heated in a reaction vessel.

In accordance with this invention, there are obtained, for example, 2-acetylresorcinol, 2-propionylresorcinol, 2-benzoylresorcinol, 2-acetyl-4-ethylresorcinol and 2-acetyl-4-acetylaminoresorcinol, in high yields and in high purity in a single step, respectively, corresponding to the starting materials as mentioned previously. The 2-acylresorcinols thus formed may be isolated and purified by a conventional method such as extraction, distillation, recrystallization or a combination thereof.

As one of important aspects of this invention, 2-acetylresorcinol in particular can be obtained by a few steps of reactions by utilizing the method of this invention as set forth hereinbefore. Namely, in accordance with this invention, there is provided a process of preparing 2-acetylresorcinol which comprises:
hydrogenating resorcinol to 1,3-cyclohexanedione;
acetylating the 2-position of the 1,3-cyclohexanedione to 2-acetyl-1,3-cyclohexanedione; and
dehydrogenating the 2-acetyl-1,3-cyclohexanedione in the presence of a dehydrogenation catalyst or a dehydrogenation agent at elevated temperatures.

The reaction process is shown below:

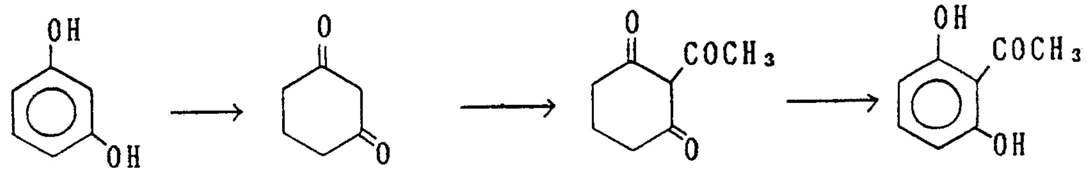

The first step is hydrogenation of resorcinol to provide 1,3-cyclohexanedione, and the step may be carried out by a known conventional manner. For example, resorcinol monosodium salt is hydrogenated with hydrogen in the presence of Raney nickel, to provide 1,3-cyclohexanedione in a high yield, as described in Organic Syntheses, Coll. Vol. 3, 278 (1955).

Acetylation of the 2-position of the 1,3-cyclohexanedione, the second step, may also be carried out by a known conventional method. For example, the 1,3-cyclohexanedione is reacted with acetic anhydride in the presence of sodium acetate, as described in J. Chem. Soc., 1953, 803. The third step has been fully described hereinbefore, and is carried out in the same manner as described hereinbefore.

This combination of the known processes and the method of dehydrogenation of 2-acetyl-1,3-cyclohexanedione of this invention makes it possible to prepare 2-acetylresorcinol in higher yields by three steps by using resorcinol as a starting material than the conventional processes.

This invention will now be described more specifically with reference to examples, however, this invention is not limited thereto.

Example 1

A solution of 22.0 g (0.2 mole) of resorcinol, 9.6 g (0.24 mole) of sodium hydroxide and 40 ml of water were placed in an autoclave together with 4.0 g of Raney nickel. The pressure in the autoclave was raised to 80 kg/cm$^2$G with hydrogen, and then the mixture was stirred at 50° C for ten hours.

After the reaction, the autoclave was depressurized and the catalyst was removed by filtration. The catalyst was thoroughly washed with a 10% aqueous solution of sodium hydroxide, and the washing was combined with the filtrate. The combined aqueous solution was adjusted to a pH of 3 with a 36 % aqueous hydrochloric acid solution and then extracted with ether. The ether solution was dried over anhydrous sodium sulfate, filtered, and the filtrate was concentrated. The resultant concentrate was recrystallized from toluene and dried in a vacuum desiccator to remove toluene, to provide 20.4 g (91.0 % yield) of 1,3-cyclohexanedione (mp. 103-104° C) as crystals.

A mixture of 11.2 g (0.1 mole) of 1,3-cyclo hexanedione, 20.5 g (0.2 mole) of acetic anhydride and 1.64 g (0.02 mole) of anhydrous sodium acetate was refluxed at 125° C for 7 hours, and then the reaction mixture was left standing to room temperature. The reaction mixture was then made neutral with 2.03 g (0.02 mole) of a 36 % aqueous hydrochloric acid solution, and excess acetic anhydride and generated acetic acid were removed by distillation with a rotary evaporator. The resultant concentrate was distilled under reduced pressure to provide 13.3 g of a fraction having a boiling point of 126-127° C/22 mmHg.

By chromatographic analysis using a 10 % PEG 20M-Chromosorb WAW DMCS column (tradename, by Chromato Kogyo K.K.), the fraction was found to be 2-acetyl-1,3-cyclohexanedione of 95.0 % purity and was found to contain 3 % by weight of 3-acetoxy-2-cyclohexenone.

To remove the by-product from the fraction, ether and a saturated aqueous solution of sodium carbonate were added to the fraction. The separated aqueous layer was made neutral with a 10 % hydrochloric acid solution, and then the mixture was extracted with ether. The ether solution was dried, and concentrated by removing the ether by distillation under reduced pressure, to provide 12.3 g (80.0 mole % yield) of 2-acetyl-1,3-cyclohexanedione of 99.9 % purity.

A solution of 1.54 g (0.01 mole) of 2-acetyl-1,3-cyclohexanedione in 10.0 g of 2-propanol was added dropwise to a mixture of 0.10 g of 5 % Pd/activated carbon and 10.0 g of tetraethylene glycol dimethyl ether at 185° C over one hour. After the addition, the reaction mixture was kept at 185° C for five hours while nitrogen was blown into the reaction mixture.

After the reaction, the reaction mixture was left standing to room temperature, and the catalyst was removed from the reaction mixture by filtration. The catalyst was fully washed with diethyl ether, and the ether washing was combined with the filtrate. The combined organic solution was extracted with water to remove the tetraethylene glycol dimethyl ether therefrom, and the ether solution was dried over anhydrous sodium sulfate.

The ether solution was filtered to remove the sodium sulfate, and then the ether was distilled off. The

resultant concentrate was recrystallized from toluene, and the crystals were dried in a vacuum desiccator to remove the toluene, to provide 1.29 g of pale yellow crystals, mp. 157-158 °C. By gas chromatographic analysis using a 3 % Silicone OV-17 chromosorb WAW DMCS column (tradename, by Gaschro Kogyo K.K.), the resultant 2-acetylresorcinol was found to be 99.7 % purity and the yield was 84.6 mole %.

Example 2

A solution of 1.54 g (0.01 mole) of 2-acetyl-1,3-cyclohexanedione in 10.0 g of 2-propanol was added dropwise to a mixture of 0.10 g of 5 % Pd/activated carbon, 1.30 g (0.011 mole) of α-methylstyrene and 10.0 g of triethylene glycol dimethyl ether at 185 °C over one hour. After the addition, the reaction mixture was kept at 185 °C for five hours while nitrogen was blown into the reaction mixture.

After the reaction, the reaction mixture was worked up in the same manner as in Example 1, to provide 2-acetylresorcinol of 99.5 % purity in 81.8 mole % yield.

Example 3

An amount of 1.85 g (0.015 mole) of nitrobenzene was used in place of α-methylstyrene, and otherwise in the same manner as in Example 2, the reaction was carried out, to provide 2-acetylresorcinol of 99.3 % purity in 79.3 mole % yield.

Example 4

Diphenyl ether was used as a solvent in place of tetraethylene glycol dimethyl ether, and otherwise in the same manner as in Example 1, the reaction was carried out.

After the reaction, the reaction mixture was left standing to room temperature, and the catalyst was removed from the reaction mixture by filtration. The catalyst was fully washed with diethyl ether, and the ether washing was combined with the filtrate. The combined organic solution was concentrated, and the concentrate was recrystallized from toluene, to provide 2-acetylresorcinol of 99.6 % purity in 69.2 mole % yield.

Example 5

An amount of 1.68 g (0.01 mole) of 2-propionyl-1,3-cyclohexanedione was used in place of 2-acetyl-1,3-cyclohexanedione, and otherwise in the same manner as in Example 1, the reaction was carried out, to provide 2-propionylresorcinol of 99.6 % purity in 83.0 mole % yield.

Example 6

An amount of 2.16 g (0.01 mole) of 2-benzoyl-1,3-cyclohexanedione was used in place of 2-acetyl-1,3-cyclohexanedione, and otherwise in the same manner as in Example 1, the reaction was carried out, to provide 2-benzoylresorcinol of 99.3 % purity in 63.2 mole % yield.

Example 7

An amount of 1.82 g (0.01 mole) of 2-acetyl-4-ethyl-1,3-cyclohexanedione was used in place of 2-acetyl-1,3- cyclohexanedione, and otherwise in the same manner as in Example 1, the reaction was carried out, to provide 2-acetyl-4-ethylresorcinol of 99.8 % purity in 83.0 mole % yield.

Example 8

An amount of 2.11 g (0.01 mole) of 2-acetyl-4-acetylamino-1,3-cyclohexanedione was used in place of

2-acetyl-1,3-cyclohexanedione, and otherwise in the same manner as in Example 1, the reaction was carried out, to provide 2-acetyl-4-acetylaminoresorcinol of 99.4 % purity in 72.3 mole % yield.

Example 9

As a catalyst 5 % Rh/alumina was used in place of 5 % Pd/activated carbon and otherwise in the same manner as in Example 1, the reaction was carried out, to provide 2-acetylresorcinol of 99.2 % purity in 72.0 mole % yield.

Example 10

As a catalyst 5 % Pt/activated carbon was used in place of 5 % Pd/activated carbon and otherwise in the same manner as in Example 1, the reaction mixture was carried out, to provide 2-acetylresorcinol of 99.3 % purity in 73.5 mole % yield.

Example 11

A mixture of 1.54 g (0.01 mole) of 2-acetyl-1,3-cyclohexanedione and 0.32 g (0.01 gram atom) of sulfur powder was heated at 250° C for two hours. The reaction mixture was then dissolved in toluene, treated with activated carbon, and hot filtered. The filtrate was left standing to cool, whereupon crystals deposited. The crystals were separated by filtration and dried in a vacuum desiccator to remove toluene, to provide 0.91 g (59 mole %) of 2-acetylresorcinol of 99.0 % purity.

Reference Example 1

To a mixture of 5.5 g (0.05 mole) of resorcinol and 50 g of o-dichlorobenzene were added 7.0 g (0.0525 mole) of anhydrous aluminum chloride, and the mixture was stirred at 80° C for two hours. Then 4.71 g (0.06 mole) of acetyl chloride was added dropwise to the mixture, and heated at 30° C and then at 150° C over a period of half an hour, respectively.

After standing the reaction mixture to room temperature, ether and a 10 % aqueous hydrochloric acid solution were added to the reaction mixture, and layer separation was effected. The ether layer was washed with water, dried and concentrated.

By gas chromatographic analysis, the conversion of resorcinol was found to be 66 mole %, and the yield of 2-acetylresorcinol was 0.3 mole %. Main reaction product was 4-acetylresorcinol and the yield was 49 mole %.

Reference Example 2

An amount of 14.0 g (0.105 mole) of anhydrous aluminum chloride was used, and otherwise in the manner as in Reference Example 1, the reaction was carried out. As results, the conversion of resorcinol was found to be 70 mole %, and the yield of 2-acetylresorcinol was 0.9 mole %. Main reaction product was 4-acetylresorcinol and the yield was 57 mole %.

## Claims

1. A method of preparing a 2-acylresorcinol which comprises dehydrogenating a 2-acyl-1,3-cyclohexanedione of the general formula

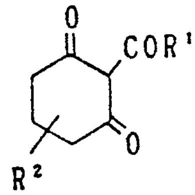

wherein $R^1$ represents an alkyl, aralkyl or aryl group, and $R^Z$ represents hydrogen, or an alkyl, alkoxy or acylamino group, in the presence of a dehydrogenation catalyst or a dehydrogenation agent, at an elevated temperature, to provide a 2-acylresorcinol of the general formula

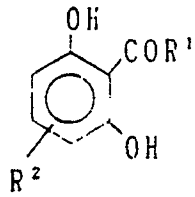

wherein $R^1$ and $R^Z$ are as hereinbefore defined.

2. A method according to claim 1, wherein the dehydrogenation catalyst is a Group VIII metal catalyst, optionally supported on a carrier.

3. A method according to claim 2 wherein the Group VIII metal is Ni, Rh, Pd or Ir.

4. A method according to claim 1 wherein the dehydrogenation catalyst is Pd supported on activated carbon.

5. A method according to claim 1 wherein the dehydrogenation catalyst is Rh supported on alumina.

6. A method according to claim 1 wherein the dehydrogenation catalyst is Pt supported on activated carbon.

7. A method according to claim 1 wherein the dehydrogenation agent is sulfur or selenium.

8. A method according to any one of the preceding claims wherein the dehydrogenation is carried out at a temperature of 100 to 250°C in the presence of an inert organic solvent.

9. A method according to any one of the preceding claims wherein hydrogen generated during the dehydrogenation reaction is removed from the reaction mixture.

10. A method according to any one of the preceding claims wherein said 2-acyl-1,3-cyclohexanedione is 2-acetyl-1,3-cyclohexanedione which has been prepared by hydrogenating resorcinol to 1,3-cyclohexanedione and acetylating the 2-position of the 1,3-cyclohexanedione.